# EUROPÄISCHE PATENTSCHRIFT

(11) **EP 3 568 171 B1**
(45) Veröffentlichungstag und Bekanntmachung des Hinweises auf die Patenterteilung: **28.02.2024**
(21) Anmeldenummer: 18700989.9
(22) Anmeldetag: 11.01.2018
(51) Int. Cl.: A61M 1/16, A61M 1/28

(54) **VORRICHTUNG UND VERFAHREN ZUR HERSTELLUNG GEBRAUCHSFERTIGER LÖSUNGEN FÜR DIE PERITONEALDIALYSE**
APPARATUS AND METHOD FOR PRODUCING READY-TO-USE SOLUTIONS FOR PERITONEAL DIALYSIS
PROCÉDÉ ET DISPOSITIF DE RÉALISATION DE SOLUTIONS PRÊTES À L'EMPLOI POUR LA DIALYSE PÉRITONÉALE

(30) Priorität: 11.01.2017 DE 102017000194; 20.01.2017 DE 102017000533
(43) Veröffentlichungstag der Anmeldung: 20.11.2019
(73) Patentinhaber: Fresenius Medical Care Deutschland GmbH, 61352 Bad Homburg (DE)
(72) Erfinder: WEISS, Stefan, 61352 Bad Homburg (DE)
(74) Vertreter: Herrmann, Uwe
(86) Internationale Anmeldenummer: PCT/EP2018/050673
(87) Internationale Veröffentlichungsnummer: WO 2018/130617

(56) Entgegenhaltungen:
- WO-A1-00/57833
- WO-A1-00/57935
- WO-A1-97/05851
- WO-A1-97/41902
- WO-A2-2012/129501
- GB-A- 2 091 126

## Beschreibung

Die Erfindung betrifft eine Vorrichtung, ein Verfahren und ein System zur Herstellung gebrauchsfertiger Lösungen für die Peritonealdialyse.

Lösungen für die Peritonealdialyse werden im Stand der Technik oftmals großtechnisch hergestellt und dem Patienten gebrauchsfertig zur Verfügung gestellt. Ferner ist es bekannt, Mehrkammerbeutel mit den Einzelbestandteilen derartiger Lösungen zur Verfügung zu stellen, um die Einzelbestandteile vor Ort vermischen zu können. In beiden Fällen umfasst die zur Verfügung gestellte Einheit alle Inhaltsstoffe der gebrauchsfertigen Lösung, wobei Wasser ein Hauptbestandteil davon ist.

Nachteilig an diesen Formen der Bereitstellung ist, dass das Versenden und die Lagerung von gebrauchsfertigen Lösungen bzw. von Mehrkammerbeuteln mit allen Einzelbestandteilen derartiger Lösungen aufwendig sind. In der WO 2013/055283 A1 wurde angedacht, dem Patienten nur noch Konzentrate zur Verfügung zu stellen und die gebrauchsfertige Lösung durch Zusatz von Leitungswasser am Ort der Anwendung herzustellen. Um dabei am Anwendungsort auf steriles Wasser zurückgreifen zu können, ist vorgesehen, dass Leitungswasser vor dem Mischen mit dem Konzentrat mit einer Umkehrosmoseanlage aufbereitet wird und die hergestellte gebrauchsfertige Lösung vor Applikation noch einen Sterilfilter passiert. Weiteren Stand der Technik stellen die Druckschriften WO00/57935A, GB2091126A und WO00/57833A1 dar.

Aufgabe der Erfindung ist es, gebrauchsfertige Lösungen aus lediglich Konzentraten am Anwendungsort herzustellen, bei denen eine Sterilität der Lösung in besserem Maße gesichert ist als in den bekannten Lösungen.

Vor diesem Hintergrund betrifft die Erfindung eine Vorrichtung zur Herstellung gebrauchsfertiger Lösungen für die Peritonealdialyse, die ein Sterilisationsfach, eine zum Sterilisationsfach führende Befüllleitung mit einem an deren fachseitigem Ende angeordneten Konnektor, eine in der Befüllleitung angeordnete Wasseraufbereitungsanlage und eine Versiegelungsanlage aufweist, wobei die Versiegelungsanlage und der Konnektor zum Anschluss eines Disposables innerhalb des Sterilisationsfachs angeordnet sind und die Wasseraufbereitungsanlage außerhalb des Sterilisationsfachs angeordnet ist.

Im Betrieb einer derartigen Vorrichtung kann ein Disposable oder zumindest ein Lösungsbeutel eines Disposables in das Sterilisationsfach eingebracht werden. Der Konnektor kann beispielsweise so ausgebildet sein, dass eine reversibel lösbare mechanische Verbindung mit einem geeigneten Gegenkonnektor an einem Disposable erfolgen kann. Er kann eine männliche oder weibliche Schnittstelle, eine Rastnase oder Rastaufnahme, ein Außengewinde oder Innengewinde oder dergleichen umfassen. Der Konnektor kann als Luer-Verbindung ausgeführt sein. Die Vorrichtung weist eine Zugangsöffnung auf, durch die ein Disposable in das Sterilisationsfach eingelegt werden kann. Vorzugsweise ist ein Abschlusselement wie beispielsweise eine Deckklappe vorhanden, mit dem die Zugangsöffnung verschlossen werden kann.

In einer Ausführungsform ist vorgesehen, dass es sich bei dem Sterilisationsfach um ein Heizfach und vorzugsweise um einen Autoklaven handelt. Im Falle eines Heizfachs umfasst die Vorrichtung eine dazugehörige Heizung und ist so ausgebildet, dass im Heizfach eine Temperatur von 60°C bis 150°C, vorzugsweise von 80°C bis 120°C eingestellt werden kann. Bei Verwendung eines Autoklaven kann eine Lösung, die sich in einem Behältnis befindet, das im Heizfach aufgenommen ist, unter erhöhtem Druck erwärmt werden. Die Erwärmung kann isochor erfolgen oder es kann sogar darüber hinaus zusätzlicher Druck aufgebaut werden. So wird im Falle einer beispielsweise carbonatgepufferten Lösung ein Ausgasen vermieden. Ferner können höhere Temperaturen erreicht werden, bevor der Siedepunkt der Lösung erreicht ist.

In einer Ausführungsform umfasst die Vorrichtung eine Strahlenquelle, mit der ein im Sterilisationsfach befindliches Disposable bestrahlt werden kann. Eine Strahlenquelle kann zusätzlich oder statt einer Heizung vorhanden sein. Bei der Strahlenquelle kann es sich um eine Quelle für UV-Licht handeln.

In einer Ausführungsform ist vorgesehen, dass die Versiegelungsanlage eine beheizbare Schweißschiene und/oder einen Ultraschallgenerator aufweist. Versiegelungsanlagen mit beheizbaren Schweißschienen sind beispielsweise aus Folienschweißgeräten und Vakuumiergeräten bekannt. Mit einer derartigen Anlage kann eine als Kunststoffschlauch ausgebildete Zulaufleitung eines in der Kammer befindlichen Disposables verschlossen werden. Auch Ultraschallschweißen kann geeignet sein, um eine beispielsweise aus einem Kunststoffschlauch bestehende Zulaufleitung eines Beutels verschweißen zu können.

In einer Ausführungsform ist vorgesehen, dass die Befüllleitung an ihrem gegenüberliegenden Ende eine für den Benutzer zugängliche Schnittstelle zum Anschluss an eine Wasserversorgung aufweist.

In einer Ausführungsform ist vorgesehen, dass die Wasseraufbereitungsanlage mindestens einen Filter, vorzugsweise eine Umkehrosmoseeinheit umfasst. Daneben kann die Wasseraufbereitungsanlage auch adsorptive Elemente sowie Sterilfilter enthalten. Die Anlage kann auch Destillen aufweisen.

Vor dem eingangs genannten Hintergrund betrifft die Offenbarung ferner ein Disposable zur Herstellung gebrauchsfertiger Lösungen für die Peritonealdialyse in einer erfindungsgemäßen Vorrichtung, wobei das Disposable ein Behältnis mit wenigstens einer Kammer zur Aufnahme von Wasser aufweist, und wobei das Disposable eine in die Kammer führende Zulaufleitung aufweist, die einen Gegenkonnektor zur lösbaren Verbindung mit dem Konnektor der Vorrichtung umfasst. Der Gegenkonnektor kann beispielsweise so ausgebildet sein, dass eine reversibel lösbare mechanische Verbindung mit dem Konnektor der Vorrichtung erfolgen kann. Er kann eine weibliche oder männliche Schnittstelle, eine Rastaufnahme oder Rastnase, ein Innengewinde oder Außengewinde oder dergleichen umfassen.

In einer Variante ist vorgesehen, dass das Behältnis in derselben oder in einer weiteren Kammer ein festes oder flüssiges Konzentrat für eine Peritonealdialyselösung beinhaltet. Bei dem Behältnis kann es sich beispielsweise um ein Behältnis mit mehreren Kammern, wie ein Mehrkammerbeutel, handeln, wobei zwei Kammern durch eine Schweißnaht voneinander getrennt sind, die durch Druckausübung auf eine der daran anliegenden Kammern geöffnet werden können. Beispielsweise kann es sich um einen Zwei- oder Dreikammerbeutel handeln, wobei eine Kammer mit der Zulaufleitung verbunden ist und mit Wasser befüllt werden kann, und wobei die andere Kammer oder die anderen Kammern das Lösungskonzentrat oder jeweils Teile des Lösungskonzentrats enthalten.

In einer Variante ist vorgesehen, dass das Disposable ferner ein Schlauchset zur Verbindung des Behältnisses mit einem Patienten aufweist, wobei das Schlauchset und das Behältnis vorzugsweise untrennbar miteinander verbunden sind. Das Schlauchset kann beispielsweise einstückig mit dem Behältnis gefertigt sein. Das Disposable kann insgesamt aus Kunststoff gefertigt sein. Bei der Zulaufleitung kann es sich um einen Kunststoffschlauch handeln. Vorzugsweise ist das Kunststoffmaterial thermoplastisch, sodass es thermisch verschweißt werden kann. Im Gegensatz zu den derzeit verwendeten Disposables ist also das Behältnis bereits Bestandteil des geschlossenen Systems und muss nicht mehr an ein Schlauchset konnektiert werden.

Des Weiteren betrifft die Erfindung ein Verfahren zur Herstellung gebrauchsfertiger Lösungen für die Peritonealdialyse, wobei ein offenbarungsgemäßes Disposable in das Sterilisationsfach einer erfindungsgemäßen Vorrichtung eingelegt wird, wobei der Konnektor der Vorrichtung mit dem Gegenkonnektor des Disposables verbunden wird, wobei die Kammer des Disposables durch die Befüllleitung der Vorrichtung und die Zulaufleitung des Disposables mit Wasser befüllt wird, wobei die Zulaufleitung des Disposables mit der Versiegelungsanlage der Vorrichtung versiegelt wird, und wobei das befüllte Disposable durch Erhöhung der Temperatur im Sterilisierungsfach sterilisiert wird.

Die Sterilisation kann beispielsweise durch Hitze und/oder Bestrahlung erfolgen. Eine Hitzesterilisation kann beispielsweise bei einer Temperatur von 60°C bis 150°C, vorzugsweise von 80°C bis 120°C erfolgen. Sie kann unter erhöhtem Druck von beispielsweise größer 1,2 bar oder größer 1,5 bar durchgeführt werden. Temperatur und Druck können beispielsweise für einen Zeitraum von mehr als 10 Minuten oder mehr als 20 Minuten gehalten werden, um eine gleichmäßige Temperierung der gesamten Lösung zu erreichen.

Die Zulaufleitung des Disposables, bei der es sich beispielsweise um einen Kunststoffschlauch handeln kann, kann beispielsweise durch thermisches Schweißen und/oder Ultraschallschweißen versiegelt werden, sodass während der Sterilisation kein Fluid mehr aus der Kammer des Disposables austreten oder in diese eintreten kann. Das abgeschweißte Disposable bildet ein geschlossenes System, das im Wesentlichen gas- und flüssigkeitsdicht ist.

Die Vorrichtung muss vor dem Befüllen der Kammer des Behältnisses mit Wasser an eine Wasserleitung angeschlossen werden, beispielsweise an eine normale Trinkwasserleitung eines Haushalts. Vorzugsweise erfolgt dieser Anschluss bereits vor Einlegen des Disposables bzw. zumindest vor Verbinden des Konnektors der Vorrichtung mit dem Gegenkonnektor des Disposables, sodass die Befüllleitung mit Wasser durchspült und so entgast werden kann.

Beim Durchlaufen der Befülleitung wird das Wasser in der Wasseraufbereitungsanlage filtriert und vorzugsweise deionisiert und/oder vorsterilisiert. Dabei kann eine Umkehrosmose durchgeführt werden. Ferner ist möglich, dass eine Bestrahlung erfolgt und/oder ein Sterilfilter durchlaufen wird.

Nach der Befüllung mit Leitungswasser kann die Zulaufleitung also zugeschweißt und so ein geschlossenes System hergestellt werden. Das so hergestellte System wird in der Sterilisationskammer, bei der es sich um einen Autoklaven handeln kann, am Anwendungsort sterilisiert und vorzugsweise hitzesterilisiert.

Im Rahmen des erfindungsgemäßen Verfahrens ist also vorgesehen, sterile Peritonealdialyselösungen batchweise am Anwendungsort herzustellen. Es wird eine gebrauchsfertige Peritonealdialyselösung am Anwendungsort in einem Disposable gemischt, bei dem es sich vorzugsweise um ein vorgefertigtes Administrationsset bestehend aus Behältnis und Schläuchen handelt, sodass die gebrauchsfertige Lösung in einem Behältnis vorliegt. Alternativ können auch mehrere Lösungen getrennt in einem Mehrkammer-Beutelsystem vorgelegt werden, die erst vor Gebrauch zur gebrauchsfertigen Lösung gemischt werden. Die Befüllung und das Mischen der Lösung können dabei mit Leitungswasser einerseits und aus Konzentratbehältern andererseits erfolgen. Bevorzugt ist jedoch dass die Konzentrate in flüssiger oder trockener Form bereits in dem Lösungsbeutel vorgelegt sind. Die vorgelegten Konzentrate können ggf. in einer anderen Kammer vorgelegt werden, z.B. in einem Mehrkammerbeutel mit Peelnaht, wie er beispielsweise in der WO 2011/073274 A1 offenbart wird.

In einer Ausführungsform ist vorgesehen, die im Rahmen des erfindungsgemäßen Verfahrens im Disposable hergestellte Lösung vor der Applikation an einen Patienten zu analysieren, beispielsweise mittels Leitfähigkeitsmessung und/oder pH-Messung. Auf diese Weise kann sichergestellt werden, dass ein Konzentrat mit der richtigen Menge Wasser verdünnt wurde und somit eine zu verabreichende Lösung mit der richtigen Konzentration hergestellt wurde.

Letztlich betrifft die Erfindung ein System umfassend eine erfindungsgemäße Vorrichtung und ein in das Sterilisationsfach dieser Vorrichtung eingelegtes offenbarungsgemäßes Disposable, wobei der Konnektor der Vorrichtung mit dem Gegenkonnektor des Disposables verbunden ist.

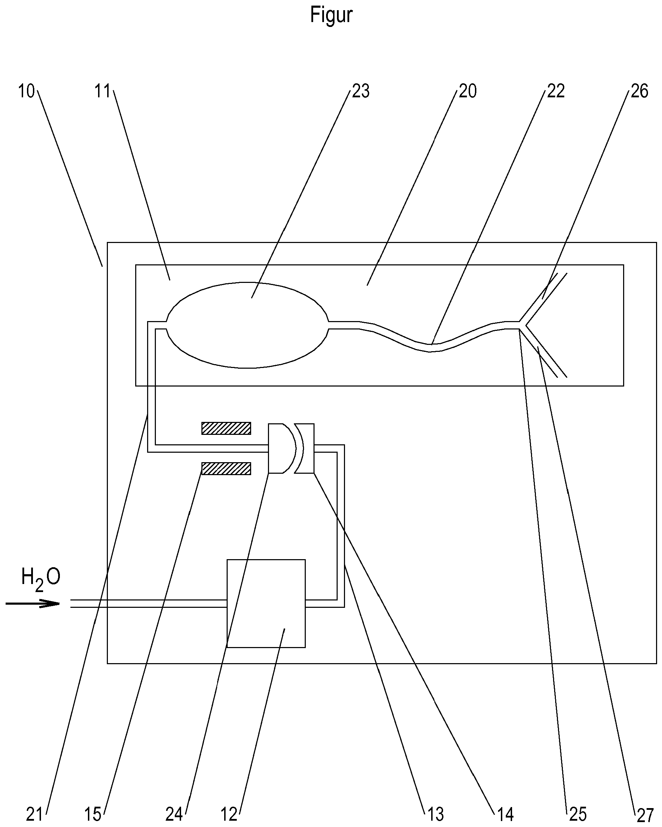

Weitere Einzelheiten und Vorteile der Erfindung ergeben sich aus dem nachfolgend anhand der Figur beschriebenen Ausführungsbeispiel.

Die Figur zeigt eine schematische Darstellung einer erfindungsgemäßen Vorrichtung 10 mit einem darin aufgenommenen offenbarungsgemäßen Disposable 20, das als Beutel-Schlauch-System ausgebildet ist.

Die Vorrichtung 10 umfasst einen Autoklaven 11, in dem das Disposable 20 angeordnet ist. Ferner umfasst die Vorrichtung eine Wasseraufbereitungsanlage 12, die eine Umkehrosmoseeinheit beinhaltet, sowie eine Befüllleitung 13, die von einer außerhalb der Vorrichtung 10 befindlichen Wasserquelle bis zu einem Konnektor 14 im oder nahe dem Autoklaven 11 reicht. Der Konnektor 14 dient dem Anschluss der Befüllleitung 13 an das Disposable 20. Die Aufbereitungsanlage 12 befindet sich an der Befüllleitung 13, sodass Wasser am Weg von der Wasserquelle zum Konnektor 14 durch die Wasseraufbereitungsanlage 12 laufen muss. Dem Konnektor 14 vorgelagert befindet sich eine Versiegelungsanlage 15 mit zwei Heizschienen zum Verschweißen einer Zulaufleitung 21 des Disposables 20 und zur Herstellung eines geschlossenen Systems innerhalb des Disposables 20. Der Konnektor 14 und die Versiegelungsanlage 15 können entweder im Autoklaven 11 oder neben dem Autoklaven 11 angeordnet sein. Bevorzugt ist die Versiegelungsanlage innerhalb des Sterilisationsfachs angeordnet. Auf diese Weise liegen alle Bestandteile des geschlossenen Systems innerhalb des Sterilisationsfachs.

Das Disposable 20 umfasst neben der Zulaufleitung 21 ein Schlauchset 22 und einen mit der Zulaufleitung 21 und dem Schlauchset 22 verbundenen Behältnis 23. Durch die vorgefertigte Verbindung von Schlauchset 22 und Behältnis 23 bietet dieses Disposable 20 im Vergleich zu herkömmlichen Disposables eine Kostenersparnis, denn es entfallen ansonsten notwendige separate Konnektoren. Die Zulaufleitung 21 weist an deren dem Behältnis 23 abgewandtem Ende einen Gegenkonnektor 24 auf, der mit dem Konnektor 14 der Vorrichtung lösbar mechanisch verbunden ist. Das Schlauchset 22 umfasst eine Y-Verzweigung 25 mit einer Leitung 26 zum Anschluss an einen PD-Katheter und mit einer weiteren Leitung 27 zum Abführen von verbrauchtem Konzentrat. Die Leitung 27 kann flüssigkeitsdicht mit einem Behälter, vorzugsweise Beutel, zur Aufnahme des verbrauchten Dialysats verbunden sein.

Die Leitung 22, die Verzweigung 25 sowie die Leitungen 26 und 27 befinden sich ebenfalls innerhalb der Vorrichtung 10 bzw. innerhalb des Sterilisationsfachs, wie dies aus der Figur hervorgeht.

Das Füllvolumen des Behältnis 23 beträgt beispielsweise zwei bis fünf Liter, wie dies auch im Stand der Technik üblich ist. Weil das Disposable 20 am Anwendungsort befüllt werden und ein Transport somit entfallen kann, ist aber auch denkbar, statt mehrerer Lösungsbeutel mit zwei bis fünf Litern Inhalt einen wesentlich größeren Behältnis mit einem Füllvolumen von beispielsweise bis zu dreißig Litern zu nutzen. Zusätzlich zu einem solchen großen Lösungsbeutel kann in der Therapie ein kleinerer Last Bag zur Anwendung kommen.

In dem Behältnis kann ein Dialysekonzentrat vorgelegt sein. Bevorzugt ist das Konzentrat in einer Kammer des Beutels vorgelegt wobei die Kammer durch einen brechbaren Verschluss in die Hauptkammer des Behältnis entleert werden kann. Bei dem brechbaren Verschluss kann es sich um eine Peelnaht handeln.

Ferner enthält das Disposable vorzugsweise einen brechbaren Verschluss vor oder in der Ablaufleitung 22. Der Verschluss kann beispielsweise als Sicherheitspeelnaht ausgeführt sein, die eine Auslaufkammer definiert oder als ein Brechkonus in der Ablaufleitung. Auf diese Weise kann sichergestellt werden, dass eine vollständige Mischung des Konzentrats mit dem Wasser erfolgt bevor der Lösungsbeutel dem Patienten verabreicht wird.

In der Anwendung wird das Disposable 20 in den Autoklaven 11 eingelegt und die Zulaufleitung 21 des Disposables wird mit der Befüllleitung 13 der Vorrichtung 10 verbunden. Genauer gesagt wird der Konnektor 14 mit dem Gegenkonnektor 24 verbunden. Die Verbindung von Konnektor 14 und Gegenkonnektor 24 erfolgt vorzugsweise im Inneren der Vorrichtung, wobei der Konnektor 14 für den Benutzer beispielsweise durch den geöffneten Autoklaven 11 zugänglich sein kann. Bei der Verbindung wird gleichzeitig die Zulaufleitung 21 des Disposables 20 zwischen die Heizschienen der Versiegelungsanlage 15 eingelegt.

Die Befüllung des Behältnis 23, das Verschweißen der Zulaufleitung 21 und das Erhitzen und Unterdrucksetzen im Autoklaven 11 zur Hitzesterilisation führt das Gerät nach Benutzereingabe automatisiert durch. So kann beispielsweise am Abend das Disposable 20 eingelegt werden, der Prozess gestartet werden und am nächsten Morgen das Disposable 20 mit einer gebrauchsfertigen Peritonealdialyselösung entnommen werden.

Nach der Hitzesterilisation und vor dem Einsatz muss die Lösung zunächst eine einsatzfähige Temperatur erreichen. Um dies bei einer Sterilisierung über Nacht zu erreichen, kann vorgesehen sein, dass die Vorrichtung den Autoklaven 11 nach der Hitzesterilisation automatisiert mit Umgebungsluft belüftet, um eine Abkühlung zu erreichen.

Das beschriebene Verfahren ist zur Herstellung von Lösungen sowohl für Continuous Ambulatory Peritoneal Dialysis (CAPD) als auch für Automated Peritoneal Dialysis (APD) geeignet.

Für einen APD-Betrieb kann das Disposable 20 beispielsweise aus dem Autoklaven 11 entnommen und in bekannter Weise in einem APD-Gerät genutzt werden. Alternativ kann die Vorrichtung eine zusätzliche Leitung aufweisen, die direkt mit einem APD-Gerät verbunden werden kann. In einem solchen Fall muss das Disposable 20 für die Therapie nicht aus dem Autoklaven 11 entnommen werden, sondern die Dialyselösung kann direkt aus dem noch im Autoklaven 11 vorliegenden gefüllten Disposable 20 an das APD-Gerät gespeist werden. Dabei kann der Autoklav 11 auch als Heizung für die Temperierung der Dialyselösung in der Therapie genutzt werden, sodass im eigentlichen APD-Gerät keine Heizung mehr vorhanden sein muss.

In einer Variante kann auch vorgesehen sein, dass ein APD-Gerät in der Vorrichtung 11 integriert ist, beispielsweise ein gravimetrisches APD-Gerät. In einem solchen Fall könnte die Heizung der Lösung während der Therapie durch den Autoklaven 11 erfolgen und der Autoklav 11 kann auf einer solchen Höhe installiert sein, dass eine gravimetrische APD ermöglicht wird. Ein Anheben des gefüllten Behältnis 23 auf ein für die gravimetrische APD notwendiges Level würde sich erübrigen, weil die Befüllung vor Ort erfolgen würde.

## Patentansprüche

1. Vorrichtung zur Herstellung gebrauchsfertiger Lösungen für die Peritonealdialyse,
**dadurch gekennzeichnet,**
**dass** die Vorrichtung ein Sterilisationsfach, eine zum Sterilisationsfach führende Befüllleitung mit einem an deren fachseitigem Ende angeordneten Konnektor zum Anschluss eines Disposables, eine in der Befüllleitung angeordnete Wasseraufbereitungsanlage und eine Versiegelungsanlage aufweist, wobei die Versiegelungsanlage und der Konnektor zum Anschluss eines Disposables innerhalb des Sterilisationsfachs angeordnet sind und dass die Wasseraufbereitungsanlage außerhalb des Sterilisationsfachs angeordnet ist.

2. Vorrichtung nach Anspruch 1, **dadurch gekennzeichnet, dass** es sich bei dem Sterilisationsfach um ein Heizfach und/oder um ein Bestrahlungsfach handelt.

3. Vorrichtung nach Anspruch 2, **dadurch gekennzeichnet, dass** es sich bei dem Heizfach um einen Autoklaven handelt.

4. Vorrichtung nach Anspruch 2 oder 3, **dadurch gekennzeichnet, dass** das Bestrahlungsfach eine Strahlungsquelle aufweist, die UV-Licht abgibt.

5. Vorrichtung nach einem der vorhergehenden Ansprüche, **dadurch gekennzeichnet, dass** die Versiegelungsanlage eine beheizbare Schweißschiene und/oder einen Ultraschallgenerator aufweist.

6. Vorrichtung nach einem der vorhergehenden Ansprüche, **dadurch gekennzeichnet, dass** die Befüllleitung an ihrem gegenüberliegenden Ende eine für den Benutzer zugängliche Schnittstelle zum Anschluss an eine Wasserversorgung aufweist.

7. Vorrichtung nach einem der vorhergehenden Ansprüche, **dadurch gekennzeichnet, dass** die Wasseraufbereitungsanlage eine Umkehrosmoseeinheit und/oder einen Sterilfilter umfasst.

8. Verfahren zur Herstellung gebrauchsfertiger Lösungen für die Peritonealdialyse,
**dadurch gekennzeichnet,**
**dass** ein Disposable in das Sterilisationsfach einer Vorrichtung nach einem der Ansprüche 1 bis 7 eingelegt wird, wobei das Disposable ein Behältnis mit wenigstens einer Kammer zur Aufnahme von Wasser aufweist, und das Disposable eine in die Kammer führende Zulaufleitung aufweist, die einen Gegenkonnektor zur lösbaren Verbin-dung mit dem Konnektor der Vorrichtung umfasst, dass der Konnektor der Vorrichtung mit dem Gegenkonnektor des Disposables verbunden wird, dass die Kammer des Disposables durch die Befüllleitung der Vorrichtung und die Zulaufleitung des Disposables mit Wasser befüllt wird, dass die Zulaufleitung des Disposables mit der Versiegelungsanlage der Vorrichtung versiegelt wird, und dass das befüllte Disposable durch Erhöhung der Temperatur im Sterilisierungsfach sterilisiert wird.

9. System umfassend eine Vorrichtung nach einem der Ansprüche 1 bis 7 und ein in das Sterilisationsfach dieser Vorrichtung eingelegtes Disposable, wobei das Disposable ein Behältnis mit wenigstens einer Kammer zur Aufnahme von Wasser aufweist, und das Disposable eine in die Kammer führende Zulaufleitung aufweist, die einen Gegenkonnektor zur lösbaren Verbindung mit dem Konnektor der Vorrichtung umfasst, wobei das Behältnis in derselben oder in einer weiteren Kammer ein festes oder flüssiges Konzentrat für eine Peritonealdialyselösung beinhaltet und / oder wobei das Disposable ferner ein Schlauchset zur Verbindung des Behältnisses mit einem Patienten aufweist, wobei das Schlauchset und das Behältnis vor-zugsweise untrennbar miteinander verbunden sind, und wobei der Konnektor der Vorrichtung mit dem Gegenkonnektor des Disposables verbunden ist.

## Claims

1. An apparatus for preparing ready-to-use solutions for peritoneal dialysis,
**characterized in that**
the apparatus has a sterilization compartment; a filling line leading to the sterilization compartment and having a connector arranged at the compartmentside end for connecting a disposable; a water treatment plant arranged in the filling line; and a sealing system, wherein the sealing system and the connector for connecting a disposable are arranged within the sterilization compartment; and **in that** the water treatment plant is arranged outside the sterilization compartment.

2. An apparatus in accordance with claim 1, **characterized in that** the steriliza-tion compartment is a heating compartment and/or an irradiation compart-ment.

3. An apparatus in accordance with claim 2, **characterized in that** the heating compartment is an autoclave.

4. An apparatus in accordance with claim 2 or claim 3, **characterized in that** the irradiation compartment has a radiation source that emits UV light.

5. An apparatus in accordance with one of the preceding claims, **characterized in that** the sealing system has a heatable sealing bar and/or an ultrasound generator.

6. An apparatus in accordance with one of the preceding claims, **characterized in that** the filling line has at its oppositely disposed end an interface accessible to the user for connection to a water supply.

7. An apparatus in accordance with one of the preceding claims, **characterized in that** the water treatment plant comprises a reverse osmosis unit and/or a sterile filter.

8. A method for preparing ready-to-use solutions for peritoneal dialysis,
**characterized in that**
a disposable is placed into the sterilization compartment of an apparatus in accordance with one of the claims 1 to 7; **in that** the disposable has a container having at least one chamber for receiving water; and **in that** the disposable has an inflow line leading into the chamber and comprising a mating connector for a releasable connection to the connector of the apparatus, **in that** the connector of the apparatus is connected to the mating connector of the disposable; **in that** the chamber of the disposable is filled with water through the filling line of the apparatus and through the inflow line of the disposable; **in that** the inflow line of the disposable is sealed using the sealing system of the apparatus; and **in that** the filled disposable is sterilized by elevating the temperature in the sterilization compartment.

9. A system comprising an apparatus in accordance with one of the claims 1 to 7 and a disposable placed into the sterilization compartment of said apparatus, wherein the disposable has a container having at least one chamber for receiving water; and wherein the disposable has an inflow line leading into the chamber and comprising a mating connector for a releasable connection to the connector of the apparatus, wherein the container includes a solid or liquid concentrate for a peritoneal dialysis solution in the same chamber or in a further chamber and/or wherein the disposable furthermore has a tubing set for connecting the container to a patient, with the tubing set and the container preferably being inseparably connected to one another, and wherein the connector of the apparatus is connected to the mating connector of the disposable.

## Revendications

1. Dispositif de réalisation de solutions prêtes à l'emploi pour la dialyse péritonéale,
**caractérisé en ce que**
le dispositif présente un compartiment de stérilisation, une conduite de remplissage conduisant au compartiment de stérilisation et dotée d'un connecteur qui est disposé sur son extrémité côté compartiment et destiné au raccordement d'un article à usage unique, une installation de traitement d'eau disposée dans la conduite de remplissage et une installation de scellage, l'installation de scellage et le connecteur destiné au raccordement d'un article à usage unique étant disposés à l'intérieur du compartiment de stérilisation et **en ce que** l'installation de traitement d'eau est disposée en dehors du compartiment de stérilisation.

2. Dispositif selon la revendication 1, **caractérisé en ce que** le compartiment de stérilisation est un compartiment chauffant et/ou un compartiment d'irradiation.

3. Dispositif selon la revendication 2, **caractérisé en ce que** le compartiment chauffant est un autoclave.

4. Dispositif selon la revendication 2 ou 3, **caractérisé en ce que** le compartiment d'irradiation présente une source de rayonnement qui émet de la lumière UV.

5. Dispositif selon l'une des revendications précédentes, **caractérisé en ce que** l'installation de scellage présente une barre de soudage pouvant être chauffée et/ou un générateur d'ultrasons.

6. Dispositif selon l'une des revendications précédentes, **caractérisé en ce que** la conduite de remplissage présente, sur son extrémité opposée, une interface accessible pour l'utilisateur, destinée au raccordement à une alimentation en eau.

7. Dispositif selon l'une des revendications précédentes, **caractérisé en ce que** l'installation de traitement d'eau comprend une unité d'osmose inverse et/ou un filtre stérile.

8. Procédé de réalisation de solutions prêtes à l'emploi pour la dialyse péritonéale,
**caractérisé en ce que**
un article à usage unique est placé dans le compartiment de stérilisation d'un dispositif selon l'une des revendications 1 à 7, l'article à usage unique présentant un contenant doté d'au moins une chambre destinée à recevoir de l'eau, et l'article à usage unique présentant une conduite d'alimentation conduisant dans la chambre et qui comprend un connecteur complémentaire destiné au raccordement détachable au connecteur du dispositif, **en ce que** le connecteur du dispositif est raccordé au connecteur complémentaire de l'article à usage unique, **en ce que** la chambre de l'article à usage unique est remplie d'eau par la conduite de remplissage du dispositif et la conduite d'alimentation de l'article à usage unique, **en ce que** la conduite d'alimentation de l'article à usage unique est scellée au moyen de l'installation de scellage du dispositif et **en ce que** l'article à usage unique rempli est stérilisé par augmentation de la température dans le compartiment de stérilisation.

9. Système comprenant un dispositif selon l'une des revendications 1 à 7 et un article à usage unique placé dans le compartiment de stérilisation dudit dispositif, l'article à usage unique présentant un contenant avec au moins une chambre destinée à recevoir de l'eau, et l'article à usage unique présentant une conduite d'alimentation conduisant dans la chambre, ladite conduite d'alimentation comprenant un connecteur complémentaire destiné au raccordement détachable au connecteur du dispositif, le contenant renfermant, dans la même chambre ou dans une autre chambre, un concentré solide ou liquide pour une solution de dialyse péritonéale et/ou l'article à usage unique présentant en outre un ensemble de tuyaux destiné au raccordement du contenant à un patient, l'ensemble de tuyaux et le contenant étant de préférence raccordés l'un à l'autre de manière inséparable et le connecteur du dispositif étant raccordé au connecteur complémentaire de l'article à usage unique.
